(19) Europäisches Patentamt
European Patent Office
Office européen des brevets

(11) Publication number: 0 068 866
B1

(12) EUROPEAN PATENT SPECIFICATION

(45) Date of publication of patent specification: 24.07.85

(51) Int. Cl.[4]: C 07 C 45/34, C 07 C 49/04, B 01 J 31/30

(21) Application number: 82303361.8

(22) Date of filing: 28.06.82

# (54) Method of manufacturing acetone or methyl ethyl ketone.

(30) Priority: 01.07.81 JP 101333/81

(43) Date of publication of application: 05.01.83 Bulletin 83/01

(45) Publication of the grant of the patent: 24.07.85 Bulletin 85/30

(84) Designated Contracting States: BE DE FR GB

(56) References cited:
EP-A-0 000 143
US-A-3 365 499
US-A-3 370 073
US-A-3 932 521
US-A-4 237 071
US-A-4 237 331

(73) Proprietor: TOA NENRYO KOGYO KABUSHIKI KAISHA
1-1 Hitotsubashi, 1-Chome Chiyoda-Ku
Tokyo 100 (JP)

(72) Inventor: Okumura, Yoshiharu
6-29-112, Shimoochiai 3-chome Shinjuku-ku
Tokyo (JP)
Inventor: Sakakibara, Tadamori
1902-5, Ooaza Kamekubo Ooi-machi
Iruma-gun Saitama-ken (JP)
Inventor: Kaneko, Katsumi
2174-19, Ooaza Nishitsurugaoka Ooi-machi
Iruma-gun Saitama-ken (JP)

(74) Representative: Northover, Robert Frank et al
ESSO Chemical Limited Esso Chemical Research Centre P.O. Box 1
Abingdon Oxfordshire, OX13 6BB (GB)

EP 0 068 866 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The so-called Hoechst-Wacker process which produces a carbonyl compound by the oxidation of an olefin in the presence of a palladium compound, a copper compound, a halogen and water has been known for a long time. By this process, acetaldehyde is produced from ethylene and acetone from propylene respectively on a commercial scale — see Angew. Chem. *71*, 176 (1959); Hydrocarbon Process and Petroleum Refiner, *42* (7) 149 (1963) and Hydrocarbon Processing, March 1976, page 97.

For the manufacture of a ketone from a higher olefin of at least four carbon atoms, no process has yet been brought to practical application because (1) the reaction velocity is low, particularly for the internal olefin as compared with the terminal olefin, (2) the reaction entails occurrence of such secondary products as aldehyde and chlorinated ketone, and (3) the corrosion of the reaction apparatus by hydrogen chloride is violent.

A number of attempts have been made to date with a view to eliminating some of the problems which are involved in the process for the manufacture of a ketone from a higher olefin.

For example, a method for producing methylethyl ketone with improved yield and selectivity by effecting the oxidation of n-butene, preferably 2-butene, in the presence of an aqueous solution of palladium compound and ferric sulfate (Japanese Patent Publication Nos. 2010/1971 and 21975/1972 and Japanese Patent Publication No. 15811/1972 equivalent to GB 1,259,145), a method for producing methyl ketone in improved yield by effecting the oxidation of a higher terminal olefin in the presence of a solvent comprising an oxygen-containing compound such as alcohol, polyol, sulfolan, or dimethyl formamide (Journal of Organic Chemistry, *29*, 241 (1964); ibid., *34*, 3949 (1969); and ibid., *39*, 3276 (1974), and a method which effects the oxidation at a low temperature in the presence of a surface active agent to improve the selectivity of the conversion to the ketone aimed at (Japanese Patent Application Disclosure No. 16419/1979 equivalent to U.S. Patent 4,152,354; see also U.S. Patent 4,203,927) have been known to the art.

Even by these known methods, however, the aforementioned problems have not yet been thoroughly eliminated.

US 3,932,521, US 3,365,499 and US 3,370,073 each disclose the preparation of higher ketones by the oxidation of various olefinically unsaturated compounds having at least 5 carbon atoms in the presence of a solvent such as dimethylformamide or dimethylacetamide. No indication is given for the preparation of ketones with less than 5 carbon atoms.

EP 0,000,143 and US 4,237,071 describe a variation of the Wacker process, particularly for the preparation of 2-hexanone from 1-hexene, using phase transfer techniques and the addition of a suitable surfactant such as a quaternary ammonium halide compound. US 4,237,331 describes a method of separating isobutylene from a $C_4$ stream by selectively oxidizing the linear olefins by a similar variation of the Wacker process. However, this process does not show consistent high selectivities to methyl ethyl ketone (the commercially important product) irrespective of feed.

A method of increasing the rate of the carbonylation reacting by the incorporation of an amine or a hydrogen halogenide salt thereof into a catalyst formed of a palladium compound and copper salt (Japanese Patent Publication No. 21402/1968) is also known to the art.

Although this method resorts to use of a tertiary amine such as trimethylamine, triethylamine, or other trialkylamine, or triethanolamine, the effect obtainable by the use of such a tertiary amine is not significant.

The inventors have made a diligent study for the purpose of providing a commercially useful method for the manufacture of acetone or methyl ethyl ketone by the oxidation of an olefin. They have discovered that the velocity and the selectivity of the reaction can be improved and the corrosion of the reaction apparatus can be diminished by incorporation of a specific tertiary amine into the catalyst system containing palladium, copper, and a halogen.

This invention resides in a method for the manufacture of acetone or methyl ethyl ketone by effecting the oxidation at a temperature of 40°C to 140°C of propylene or n-butene respectively in the presence of a catalyst comprising an aqueous solution containing palladium, copper, a halogen and at least one of the tertiary amines represented by the general formulas,

(1) $R^1R^2R^3N$, (2) 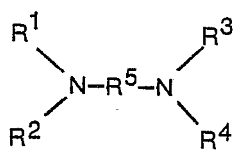 (3) 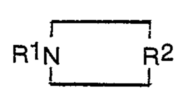 ,

and (4) 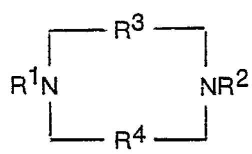

being defined as:

(1) general formula $R^1R^2R^3N$, wherein,

$R^1$ = an aryl of 6 to 20 carbon atoms, an aralkyl of 7 to 20 carbon atoms, or a substituted akyl derived from an alkyl of 1 to 16 carbon atoms by the substitution of one or more hydrogen atoms of said alkly with a halogen atom, an alkoxy of 1 to 12 carbon atoms, or an acyl of 2 to 16 carbon atoms, and

$R^2$, $R^3$ = an alkyl of 1 to 16 carbon atoms, an aryl of 6 to 20 carbon atoms, an acyl of 2 to 16 carbon atoms, an aralkyl of 7 to 20 carbon atoms, or a substituted alkyl derived from said alkyl by the substitution of one or more hydrogen atoms of said alkyl with a halogen atom, or an alkoxy of 1 to 12 carbon atoms,

provided that $R^2$ and $R^3$ may be identical, or not identical, with each other,

(2) general formula,

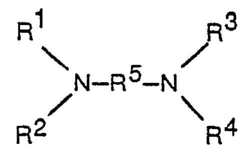

wherein,

$R^1$, $R^2$, $R^3$, $R^4$ = an alkyl of 1 to 16 carbon atoms, an aryl of 6 to 20 carbon atoms, an acyl of 2 to 16 carbon atoms, an aralkyl of 7 to 20 carbon atoms, or a substituted alkyl derived from said alkyl by the substitution of one or more hydrogen atoms of said alkyl with a halogen atom, an alkoxy of 1 to 12 carbon atoms, or an acyl of 2 to 16 carbon atoms,

provided that $R^1$, $R^2$, $R^3$, and $R^4$ may be identical, or not identical, with one another, and

$R^5$ = an alkylene of 1 to 8 carbon atoms, and arylene of 6 to 12 carbon atoms, or a group

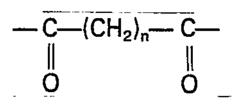

where n = 2 to 4,

(3) general formula

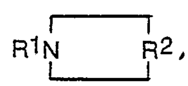

wherein,

$R^1$ = an alkyl of 1 to 16 carbon atoms, an aryl of 6 to 20 carbon atoms, an acyl of 2 to 16 carbon atoms, an aralkyl of 7 to 20 carbon atoms, or substituted alkyl derived from said alkyl by the substitution of one or more hydrogen atoms of said alkyl by a halogen atom, an alkoxy of 1 to 12 carbon atoms, or an acyl of 2 to 16 carbon atoms, and

$R^2$ =

(A) an alkylene of 3 to 6 carbon atoms,

(B) a substituted alkylene derived from said alkylene by the substitution with one or more of alkyl groups of 1 to 8 carbon atoms, aryl groups of 6 to 20 carbon atoms, aralkyl groups of 7 to 20 carbon atoms, aralkyl groups of 7 to 20 carbon atoms, or halogen atoms, and including a substituted alkylene having halogen atom substitution in substituent alkyl, aryl and aralkyl groups,

(C) a formula, $-(CH_2)_m-O-(CH_2)_n$, where m, n = 1 to 5 and (m + n) = 3 to 6, or

(D) a formula,

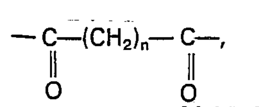

where n = 2 to 4, and

(4) general formula,

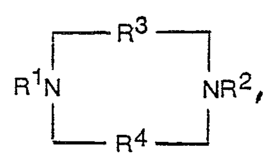

wherein,

$R^1$, $R^2$ = an alkyl of 1 to 16 carbon atoms, an aryl of 6 to 20 carbon atoms, an acyl of 2 to 16 carbon atoms, an aralkyl of 7 to 20 carbon atoms, a substituted alkyl derived from said alkyl by the substitution of

one or more hydrogen atoms of said alkyl by a halogen atom, an alkoxy of 1 to 12 carbon atoms or an acyl of 2 to 16 carbon atoms,

provided that $R^1$ and $R^2$ may be identical, or not identical with each other, and

$R^3$, $R^4$ =

(A) an alkylene of 1 to 6 carbon atoms,

(B) a substituted alkylene derived from said alkylene by the substitution of one or more hydrogen atoms of said alkylene by an alkyl of 1 to 8 carbon atoms, an aryl of 6 to 20 carbon atoms, an acyl of 2 to 16 carbon atoms, an aralkyl of 7 to 20 carbon atoms, or a halogen atom,

(C) a formula, $-(CH_2)_m-O-(CH_2)_n-$, where m, n = 1 to 5 and (m + n) = 3 to 6,

(D) a formula,

$$-(CH_2)_m-\underset{\underset{O}{\|}}{C}-(CH_2)_n-,$$

where m, n = 0 to 5 and (m + n) = 2 to 6, or

(E) a formula,

$$-\underset{\underset{O}{\|}}{C}-(CH_2)_n-\underset{\underset{O}{\|}}{C}-$$

2 to 4,

provided that $R^3$ and $R^4$ may be identical, or not identical, with each other.

Detailed Description

*Olefin*

The olefins which are usable in the present invention are propylene and n-butenes.

In the conventional method, if an iso-olefin is contained in the raw material, the iso-olefin reacts preferentially and this reaction interferes with the oxidation of the n-olefin and, moreover the reaction product of the iso-olefin is required to be removed from the main reaction product. In contrast, in accordance with the present invention, the n-olefin is selectively oxidized into a corresponding carbonyl compound. Thus, a further feature of the present invention is that a raw material containing an iso-olefin can be used safely.

The present invention, therefore, permits use of a raw material containing a terminal olefin, an internal olefin, and an iso-olefin in its unaltered form. Particularly, this invention proves advantageous for the manufacture of methylethyl ketone by using, as its raw material, the so-called $C_4$ fraction which contains isobutene, 1-butene, and 2-butene.

If the raw material contains a paraffinic hydrocarbon, the hydrocarbon has absolutely no adverse effect. When the raw material contains a diolefinic or acetylenic unsaturated hydrocarbon, however, it is desirable that such hydrocarbon be removed or converted into a monoolefin through selective hydrogenation.

*Catalyst*

The catalyst to be used in the present invention contains palladium, copper, a halogen, and a specific tertiary amine.

1) Palladium

Sources for palladium compounds. Of the palladium compounds, those preferably usable are palladium chloride, palladium bromide, palladium acetate, and palladium sulfate. Metallic palladium can be used when it is capable of forming any of the palladium compound enumerated above in situ.

2) Copper

Sources for copper are copper compounds. Of the copper compounds, those preferably usable are cupric chloride, cuprous chloride, cupric bromide, cuprous bromide, cupric sulfate, and cupric acetate. Metallic copper can be used when it is capable of forming any of the copper compounds enumerated above in situ.

3) Halogen

As regards the halogen source, when the aforementioned palladium compound and/or copper compound is used in the form of a halogenide, the halogenide can be used as a halogen source. When neither palladium halogenide or copper halogenide is used or when such a halogenide is used in an amount not enough to fill a desired halogen supply, there can be used hydrogen halogenide such as hydrogen chloride or hydrogen bromide, a halogen such as chlorine or bromine, a halogenide or alkali metal such as

sodium chloride, potassium chloride, lithium chloride, or potassium bromide, or a halogenide of alkaline earth metal such as calcium chloride, magnesium chloride, or calcium bromide.

4) Tertiary amine

The tertiary amine to be used in this invention is selected from the compounds represented by the above general formulas (1) through (4).

Examples of the compounds which are embraced by the general formula (1) given above are as follows.

Triphenylamine, diphenylmethylamine, phenyldimethylamine, tribenzylamine, dibenzylmethylamine, benzyldimethylamine, dibenzylphenylamine, diphenylbenzylamine, tri-(2-methoxyethyl)-amine, tri-(ethoxymethyl)-amine, tri-(2-chloroethyl)-amine, tri-(2-fluoroethyl)-amine, N-methylacetanilide.

Examples of the compounds which are embraced by the general formula (2) given above include, N,N,N',N'-tetraphenylethylene diamine, N,N,N',N'-tetramethyl-p-phenylene diamine, N,N,N',N'-tetrabenzylethylene diamine, N,N,N',N'-tetraacetyl-p-xylylene diamine, and N,N,N',N'-tetramethyl succinic acid amide.

Examples of the compounds which are embraced by the general formula (3) given above are as follows.

N-acetyl pyrrolidine, N-acetyl piperidine, N-phenyl pyrrolidine, N-phenyl piperidine, N-(2-chloroethyl)-pyrrolidine, N-(2-chloroethyl)-piperidine, N-acetylmethyl pyrrolidine, N-acetylmethyl piperidine, N-benzyl-pyrrolidine, N-(methoxymethyl)-pyrrolidine, N-phenyl morpholine, N-acetyl morpholine, N-methyl morpholine, N-methyl-2-phenyl pyrrolidine, N-acetyl-2-phenyl pyrrolidine, N-methyl-2-trichloromethyl pyrrolidine, N-methyl-2-trichloromethyl piperidine, N-methylsuccinic acid imide, and N,2,2,6,6,-pentamethyl piperidine.

Examples of the compounds which are embraced by the general formula (4) given above are: N,N'-diacetyl piperazine, N,N'-diphenyl piperazine, N,N'-dimethyl-2,5-dioxo piperazine, N,N'-di-(2-chloroethyl)-2, 5-diethyl piperazine, 1,7-dimethyl-1,7-diaza-4, 10-dioxocyclododecane, and 1,5-dimethyl-, 5-diaza-2, 4-dioxocycloheptane.

The catalyst to be used in the present invention contains palladium, copper, a halogen, and a specific tertiary amine. Generally, the catalyst is obtained by dissolving the compounds just mentioned in water. The palladium content in this catalyst system is 0.1 to 100 milligram atom/liter, preferably 0.5 to 20 milligram atom/liter, as palladium. The copper content is preferably 1 to 500 times, more preferably 2 to 200 times, palladium in atomic ratio. The halogen content is preferably 0.1 to 10 times, more preferably 0.5 to 3 times, copper in atomic ratio. The specific amine content is preferably such that the nitrogen content of the amine is 1 to 1,000 times, more preferably 2 to 500 times, the halogen in atomic ratio.

*Oxidation of the Olefin*

The method of this invention is accomplished by bringing the raw material containing propylene or n-butene into contact with the catalyst described above. As to the manner of this contact, either the one-step method which effects the contact in the presence of oxygen or the two-step method which comprises effecting the contact in the absence of oxygen and subsequently subjecting the catalyst to regeneration by oxidation, can be employed.

For the oxidation of the olefin, it is desirable that the olefin as the raw material and the catalyst are brought into thorough contact. Generally, such thorough contact is accomplished by a mechanical method which comprises mixing the two components and vigorously stirring the mixture.

For the purpose of facilitating the contacting of the two components, it may be effected in the presence of a solvent comprising an oxygen-containing organic compound.

Examples of solvents useful for this purpose include alcohols such as methanol and ethanol; polyols such as ethylene glycol and propylene glycol; ethers such as di-n-butyl ether and diisobutyl ether; polyalkylene glycols such as polyethylene glycol and polypropylene glycol; esters such as ethyl acetate and n-butyl acetate; ketones such as acetone and methylethyl ketone; and carboxylic acids such as formic acid and acetic acid.

The preferred conditions for the oxidation of the olefin are 50 to 110°C for reaction temperature; 1 to 50 atmospheres, more preferably 3 to 20 atmospheres for reaction pressure; and 15 minutes to 10 hours, more preferably 0.5 to 5 hours for reaction time. In the one-step process, the partial pressure of oxygen is suitably 1 to 15 atmospheres. The ratio of mixing between the olefin and the catalyst is 10 to 500 moles, preferably 20 to 300 moles, of the olefin in the two-step process, or 50 to 2,000 moles, preferably 100 to 1,000 moles, of the olefin in the one-step process, respectively per one gram atom of palladium contained in the catalyst.

The contacting of the olefin with the catalyst may be effected either batchwise or continuously and in a multiplicity of steps or in one step.

The ketone produced by the method of this invention can be isolated and recovered from the reaction system by any of the methods generally employed for treatments of this nature.

*Effect of the Invention*

The present invention possesses outstanding effects as described below.

1) the oxidation of the olefin proceeds quickly.

2) the production of an aldehyde due to the oxidation of the terminal carbon atom in an a-olefin is inhibited and the conversion to methyl ketone is promoted. Thus high selectivity to the ketone is obtained.

3) The oxidation of the internal olefin proceeds quickly and the selectivity to the ketone is high. The invention, therefore, can select the raw material from a wide range of hydrocarbons.

4) The oxidation of iso-olefin is inhibited. This means that the removal of iso-olefin from the hydrocarbon being used as the raw material, a step indispensable to the conventional method, can be omitted.

5) The formation of such secondary products as chlorinated ketone is inhibited. This means that the step for removal of such secondary products can be simplified and the purity of the produced ketone can be improved.

6) The corrosion of the reaction apparatus is diminished.

The invention will now be described in further detail with reference to the following examples which do not limit the scope of the invention.

Example 1

An autoclave of stainless steel lined with polytetrafluoroethylene was charged with 1 millimole of palladium chloride, 100 millimoles of cupric chloride, 400 millimoles of diphenylmethylamine, and 1 liter of water. With 50 millimoles of propylene incorporated under pressure, the contents of the autoclave were vigorously stirred at 70°C for one hour to effect oxidation of propylene. At the end of the reaction, the autoclave was suddenly cooled to expel the unconverted propylene.

When the reaction mixture was analyzed, the conversion of propylene was found to be 38%, the selectivity to acetone to be 99.4%, and the selectivity to chloroacetone to be 0.2%.

Example 2

1-butene as the starting olefin was oxidized by following the procedure of Example 1, except that N,N,N',N'-tetraphenylethylenediamine was used as the tertiary amine. The results are shown in Table 1.

Example 3

2-butene as the starting olefin was oxidized by following the procedure of Example 1, except that tri-(β-chloroethyl)-amine was used as the tertiary amine. The results are shown in Table 1.

Comparative Experiments 1—3

The procedures of Examples 1—3 were repeated to effect oxidation of the respective olefins, except that the respective tertiary amines were not used. The results are shown in Table 1.

TABLE 1

| Example | Olefin | Tertiary amine | Conversion of olefin (%) | Selectivity to product (%) | | |
|---|---|---|---|---|---|---|
| | | | | Ketone | Aldehyde | Chlorinated ketone |
| Example 1 | Propylene | Diphenylmethylamine | 38 | 99.4 1) | 0.4 3) | 0.2 |
| ,, 2 | 1-Butene | N,N,N',N'-tetraphenylethylenediamine | 27 | 99.7 2) | 0.5 4) | 0.1 5) |
| ,, 3 | 2-Butene | Tri-(β-chloroethyl)-amine | 18 | 99.7 2) | 0.1 4) | 0.2 5) |
| Comparative Experiment 1 | Propylene | — | 25 | 90 1) | 8 3) | 2 |
| ,, 2 | 1-Butene | — | 14 | 87 2) | 10 4) | 3 5) |
| ,, 3 | 2-Butene | — | 9 | 92 2) | 5 4) | 3 5) |

Note) 1) Acetone

2) Methylethyl ketone

3) Propion aldehyde

4) 1-Butanal

5) Total of 1-chloro-2-butanone and 3-chloro-2-butanone

## Example 4

The same autoclave that was used in Example 1 was charged with 2 millimoles of palladium chloride, 20 millimoles of cupric chloride, 50 millimoles of tribenzylamine, and 1 liter of water. With 0.5 mole of propylene incorporated under pressure and oxygen further incorporated under pressure to a partial pressure of 5 atmospheres, the contents of the autoclave were vigorously stirred to effect the reaction of propylene with oxygen. At the end of the reaction, the reaction mixture was analyzed. The results are shown in Table 2.

To determine the corroding property of the catalyst, the oxidation was carried out in the presence of the carbon steel piece 2.0 g in weight and 35 $cm^2$ in surface area. At the end of the oxidation, the carbon steel piece was weighed to find the loss due to corrosion. The results are also shown in Table 2.

## Examples 5—6

The procedure of Example 4 was repeated, except that the olefin and the tertiary amine were varied as shown in Table 2. The results are shown in Table 2.

## Comparative Experiments 4—6

The procedures of Examples 4—6 were repeated, except that the respective tertiary amines were not used. The results are shown in Table 2.

## Comparative Experiments 7 and 8

2-butene was oxidized by following the procedure of Example 6, except that the tertiary amine used in Example 6 was varied to the tertiary amines shown in Table 2. The results are shown in Table 2.

TABLE 2

| Example | Olefin | Tertiary amine | | Conversion (%) | Selectivity (%) | | | Loss of test piece (g) |
|---|---|---|---|---|---|---|---|---|
| | | Name | Amount used (millimoles) | | Ketone | Aldehyde | Chlorinated ketone | |
| Example 4 | Propylene | Tribenzylamine | 50 | 39 | 98.6[1] | 1.2[7] | 0.2 | 0.01 |
| ,, 5 | 1-Butene | N-phenyl piperidine | 60 | 16 | 99.0[2] | 0.9[8] | 0.1[11] | <0.01 |
| ,, 6 | 2-Butene | N,N′-diphenyl piperazine | 80 | 13 | 99.9[2] | <0.1[8] | 0.1[11] | <0.01 |
| Comparative Experiment 4 | Propylene | – | – | 16 | 91[1] | 7[7] | 2 | 0.26 |
| ,, 5 | 1-Butene | – | – | 8.1 | 89[2] | 8[8] | 3[11] | 0.28 |
| ,, 6 | 2-Butene | – | – | 4.2 | 94[2] | 5[8] | 1[11] | 0.25 |
| ,, 7 | 2-Butene | Triethylamine | 50 | 0.5 | – | – | – | <0.01 |
| ,, 8 | 2-Butene | Tri-n-butylamine | 50 | 0.2 | – | – | – | <0.01 |

(Note) 1) Acetone
2) Methylethyl ketone
7) Propion aldehyde
8) 1-Butanal
11) Total of 1-chloro-2-butanone and 3-chloro-2-butanone

Examples 7 and 8

By following the procedure of Example 4, 0.5 mole of 2-butene and a catalyst containing 2 millimoles of a varying palladium compound and 20 millimoles of a varying copper compound both shown in Table 3 and a varying amount of tertiary amine and a varying amount of water both shown in Table 3 were brought into contact with each other under varying reaction conditions shown in Table 3 to effectoxidation of 2-butene. The results are shown in Table 3.

Examples 9—11

The procedure of Example 1 was repeated, except a varying tertiary amine was used in a varying amount both shown in Table 4 and 100 millimoles of a $C_4$ fraction consisting of 39 mole % of iso-butene, 30 mole % of 1-butene, 17 mole % of 2-butene, 8 mole % of iso-butane, and 6 mole % of n-butane was used.

The results of the oxidation are shown in Table 4.

Comparative Experiment 9

By following the procedure of Example 9, the same $C_4$ fraction was oxidized, except the tertiary amine was not used. The results are shown in Table 4.

TABLE 3

| Example | Palladium compound | Copper compound | Tertiary amine (amount used, mole) | Water (ml) | Other additive | Reaction tem-perature (°C) | Partial pressure of oxygen (atm.) | Con-version (%) | Selectivity (%) | | | | Loss of weight in test piece (g) |
|---|---|---|---|---|---|---|---|---|---|---|---|---|---|
| | | | | | | | | | Methyl-ethyl ketone | n-Butyr aldehyde | Chloro-butanone | | |
| 7 | $PdCl^2$ | $CuCl^2$ | N,N,N';N'-tetramethyl succinic acid amide (8) | 200 | – | 100 | 5 | 83 | 99.1 | 0.8 | 0.1 | | 0.01 |
| 8 | $Pd(OAc)^2$ Note 1) | $Cu(OAc)^2$ Note 2) | N,N'-diacetyl piperazine (8) | 300 | HCl (0.1 mole) | 65 | 2 | 57 | >99.9 | <0.1 | <0.1 | | <0.01 |

Note 1) Palladium acetate    Note 2) Cupric acetate

TABLE 4

| Example | Tertiary amine | | Conversion (%) | | Selectivity based on n-butene (%) | | | Selectivity based on iso-butene (%) | |
|---|---|---|---|---|---|---|---|---|---|
| | Name | Amount used (mole) | n-Buten | Iso-butene | Methyl-ethyl ketone | n-Butyr aldehyde | Chloro-butanone | Tertiary butyl alcohol | Iso-butyr aldehyde |
| Example 9 | Diphenylmethylamine | 0.4 | 21 | 1.1 | 99.3 | 0.5 | 0.2 | 50 | 50 |
| ,, 10 | Tribenzylamino | 0.5 | 24 | 0.9 | 99.5 | 0.4 | 0.1 | 40 | 60 |
| ,, 11 | N,2,2,6,6-pentamethyl piperidine | 0.6 | 23 | 1.0 | 99.4 | 0.5 | 0.1 | 50 | 50 |
| Comparative Examperiment 11 | – | | 11 | 26 | 89 | 8 | 3 | 97 | 3 |

Example 12—17

In the presence of 4 millimoles of palladium chloride, 40 millimoles of cupric chloride, 1 liter of water, and a varying amount of a varying tertiary amine both shown in Table 5, iso-butene and 1-butene or 2-butene used each in an amount of 0.5 mole were oxidized by following the procedure of Example 4. The results are shown in Table 5. In each of the examples, a carbon steel piece was used to test the catalyst for its corrosion property.

Comparative Experiments 10—13

The same oxidation of butenes as involved in Examples 12—17 was carried out by following the procedure of such examples, except that either the tertiary amines involved therein were not used or varying tertiary amines shown in Table 5 were used. The results are shown in Table 5.

TABLE 5

| Example | | Butene mixture (mole) | | | Tertiary amine (millimole) | Conversion (%) | | Selectivity based on n-butene (%) | | | Selectivity based on iso-butene (%) | | Loss of weight in test piece (g) |
|---|---|---|---|---|---|---|---|---|---|---|---|---|---|
| | | Iso-butene | 1-Butene | 2-Butene | | n-Butene | Iso-butene | Methyl-ethyl ketone | n-Butyl aldehyde | Chloro-butanone | Tertiary butyl alcohol | Isobutyl aldehyde | |
| Example | 12 | 0.5 | 0.5 | – | Diphenylmethylamine (100) | 16 | 1.2 | 99.0 | 0.9 | 0.1 | 60 | 40 | 0.01 |
| | 13 | 0.5 | 0.5 | – | N,N,N',N'-tetramethyl-p-phenylene diamine (120) | 15 | 1.0 | 99.1 | 0.8 | 0.1 | 50 | 50 | <0.01 |
| | 14 | 0.5 | 0.5 | – | N,2,2,6,6-pentamethyl piperidine (150) | 15 | 0.9 | 98.9 | 1.0 | 0.1 | 50 | 50 | <0.01 |
| | 15 | 0.5 | – | 0.5 | Diphenylmethylamine (100) | 13 | 1.0 | 99.8 | 0.1 | 0.1 | 60 | 40 | 0.01 |
| | 16 | 0.5 | – | 0.5 | N,N'-diphenyl piperazine (120) | 12 | 0.8 | 99.8 | 0.2 | <0.1 | 40 | 60 | <0.01 |
| | 17 | 0.5 | – | 0.5 | N,2,2,6,6-pentamethyl-piperidine (150) | 13 | 1.0 | 99.8 | 0.1 | 0.1 | 50 | 50 | <0.01 |
| Comparative Experiment | 10 | 0.5 | 0.5 | – | – | 9.3 | 17 | 88 | 9 | 3 | 93 | 7 | 0.26 |
| | 11 | 0.5 | – | 0.5 | – | 5.1 | 16 | 95 | 4 | 1 | 92 | 8 | 0.24 |
| | 12 | 0.5 | 0.5 | – | Triethylamine (100) | <0.1 | <0.1 | – | – | – | – | – | <0.01 |
| | 13 | 0.5 | – | 0.5 | Tri-n-butylamine (100) | <0.1 | <0.1 | – | – | – | – | – | <0.01 |

Examples 18 and 19

By following the procedure of Example 4, a catalyst containing 2 millimoles of a varying palladium compound shown in Table 6, and varying amounts of a varying copper compound shown in Table 6, a varying tertiary amine shown in Table 6, and water (with hydrogen chloride additionally used in Example 19) and 1 mole of a $C_4$ fraction consisting of 38 mole % of isobutene, 32 mole % of 1-butene, 16 mole % of 2-butene, 8 mole % of iso-butane, and 6 mole % of n-butane were held in contact with each other under the conditions shown in Table 6 for one hour. The results are shown in Table 6.

Comparative Experiment 14

The same $C_4$ fraction as used in Example 18 was oxidized by following the procedure of the example, except that N,N,N',N'-tetramethyl succinic acid amide was not used and the amounts of copper compound and water and the reaction temperature were varied as shown in Table 6. The results are shown in Table 6.

TABLE 6

| Example | | Palladium compound (milli-mole) | Copper compound (milli-mole) | Tertiary amine (mole) | Water (ml) | Reaction tem-perature (°C) | Partial pressure of oxygen (atm) | Conversion (%) | | Selectivity based on n-butene (%) | | | Selectivity based on iso-butene (%) | | Loss of weight in test piece (g) |
|---|---|---|---|---|---|---|---|---|---|---|---|---|---|---|---|
| | | | | | | | | n-Butene | Iso-butene | Methyl-ethyl ketone | n-Butyl aldehyde | Chloro-butanone | Tertiary butyl alcohol | Isobutyl aldehyde | |
| Example | 18 | $PdCl_2$ (2) | $CuCl_2$ (40) | N,N,N',N'-tetramethyl succinic acid amide (8) | 200 | 100 | 5 | 86 | 1.2 | 99.0 | 0.9 | 0.1 | 50 | 50 | 0.01 |
| Example | *19 | $Pd(OAc)_2$ | $Cu(OAc)_2$ (20) | N,N'-diacetyl piperazine (5) | 350 | 65 | 2 | 62 | 0.7 | 99.8 | 0.2 | <0.1 | 50 | 50 | <0.01 |
| Comparative Experiment | 14 | $PdCl_2$ (2) | $CuCl_2$ (20) | | 1,000 | 75 | 5 | 3.2 | 15 | 90 | 8 | 2 | 91 | 9 | 0.22 |

*) 50 millimoles of HCl added.

$Pd(OAc)_2$ – Palladium acetate

$Cu(OAc)_2$ – Cupric acetate

**Claims**

1. A method for the manufacture of acetone or methyl ethyl ketone, which comprises oxidizing at a temperature of from 40°C to 140°C propylene or n-butene respectively in the presence of a catalyst comprising an aqueous solution containing palladium, copper, a halogen, and at least one of the tertiary amines represented by the following general formulas:

(1) general formula, $R^1R^2R^3N$, wherein

$R^1$ = an aryl of 6 to 20 carbon atoms, an aralkyl of 7 to 20 carbon atoms, or a substituted alkyl comprising an alkyl of 1 to 16 carbon atoms having one or more substituents derived by the substitution of hydrogen in said alkyl with a halogen atom, an alkoxy of 1 to 12 carbon atoms, or an acyl of 2 to 16 carbon atoms, and

$R^2$, $R^3$ which may be the same or different = an alkyl of 1 to 16 carbon atoms, an aryl of 6 to 20 carbon atoms, an acyl of 2 to 16 carbon atoms or an aralkyl of 7 to 20 carbon atoms, the alkyl being optionally substituted by one or more of halogen atoms, alkoxy groups of 1 to 12 carbon atoms, and acyl groups of 2 to 16 carbon atoms,

(2) general formula,

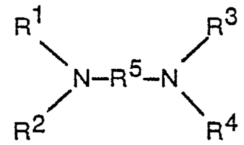

wherein,

$R^1$, $R^2$, $R^3 \cdot R^4$ which may be same or different = an alkyl of 1 to 16 carbon atoms, an aryl of 6 to 20 carbon atoms, an acyl of 2 to 16 carbon atoms or an aralkyl of 7 to 20 carbon atoms, the alkyl being optionally substituted by one or more of halogen atoms, alkoxy groups of 1 to 12 carbon atoms, and acyl groups of 2 to 16 carbon atoms, and

$R^5$ = an alkylene of 1 to 8 carbon atoms, an arylene of 6 to 12 carbon atoms, or a group

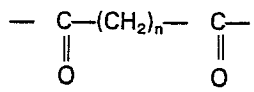

where n = 2 to 4,

(3) general formula,

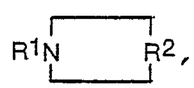

wherein,

$R^1$ = an alkyl of 1 to 16 carbon atoms, an aryl of 6 to 20 carbon atoms, an acyl of 2 to 16 carbon atoms or an aralkyl of 7 to 20 carbon atoms, the alkyl being optionally substituted by one or more of halogen atoms, alkoxy groups of 1 to 12 carbon atoms, and acyl groups of 2 to 16 carbon atoms, and

$R^2$ =

(A) an alkylene of 3 to 6 carbon atoms,

(B) an alkylene of 3 to 6 carbon atoms substituted by one or more of alkyl groups of 1 to 8 carbon atoms, aryl groups of 6 to 20 carbon atoms, aralkyl groups of 7 to 20 carbon atoms, and halogen atoms.

(C) a formula, $-(CH_2)_m-O-(CH_2)_n$, where m, n = 1 to 5 and (m + n) = 3 to 6, or

(D) a formula,

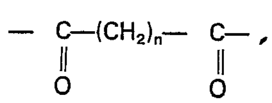

where n = 2 to 4,

(4) general formula

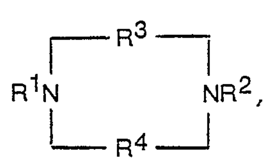

wherein,

$R^1$, $R^2$ which may be the same or different = an alkyl of 1 to 16 carbon atoms, an aryl of 6 to 20 carbon atoms, an acyl of 2 to 16 carbon atoms, an aralkyl of 7 to 20 carbon atoms, the alkyl being optionally substituted by one or more of halogen atoms, alkoxy groups of 1 o 12 carbon atoms, and acyl groups of 2 to 16 carbon atoms, and

$R^3$, $R^4$ which may be the same or different =

(A) an alkylene of 1 to 6 carbon atoms,

(B) a substituted alkylene of 1 to 6 carbon atoms substituted by one or more of alkyl groups of 1 to 8 carbon atoms, aryl groups of 6 to 20 carbon atoms, acyl groups of 2 to 16 carbon atoms, aralkyl groups of 7 to 20 carbon atoms, and halogen atoms,

(C) a formula, $-(CH_2)_m-O-(CH_2)_n-$, where m, n = 1 to 5 and (m + n) = 3 to 6,

(D) a formula,

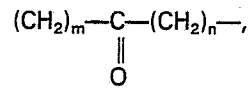

where m, n = 0 to 5, and (m + n) = 2 to 6, or

(E) a formula,

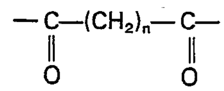

where n = 2 to 4.

2. A method according to claim 1 wherein the tertiary amine has the general formula: $R^1R^2R^3N$.

3. A method according to claim 1 wherein the tertiary amine as the general formula:

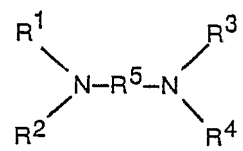

4. A method according to claim 1 wherein the tertiary amine has the general formula:

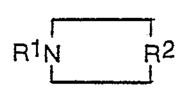

5. A method according to claim 1 wherein the tertiary amine has the general formula:

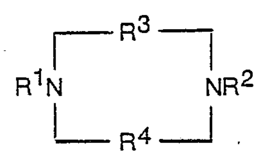

6. A method according to any of claims 1 to 5 wherein the starting material includes an iso-olefin.

**Patentansprüche**

1. Verfahren zur Herstellung von Aceton oder Methylethylketon, welches darin besteht, daß man Propylen bzw. n-Buten bei einer Temperatur von 40 bis 140°C in Gegenwart eines Katalysators oxidiert, welcher eine wäßrige Lösung mit einem Gehalt an Palladium, Kupfer, einem Halogen und mindestens einem der durch die folgenden allgemeinen Formeln dargestellten tertiären Amine umfaßt:

(1) der allgemeinen Formel $R^1R^2R^3N$, worin

$R^1$ = eine Arylgruppe mit 6 bis 20 Kohlenstoffatomen, eine Aralkylgruppe mit 7 bis 20 Kohlenstoffatomen, oder eine substituierte Alkylgruppe aus einer Alkylgruppe mit 1 bis 16 Kohlenstoffatomen mit einem oder mehreren Substituenten, die durch die Substitution von Wasserstoff in dieser Alkylgruppe durch ein Halogenatom, eine Alkoxygruppe mit 1 bis 12 Kohlenstoffatomen oder eine Acylgruppe mit 2 bis 16 Kohlenstoffatomen erhalten sind, und

$R^2$, $R^3$, welche gleich oder verschieden sein können, = eine Alkylgruppe mit 1 bis 16 Kohlenstoffatomen, eine Arylgruppe mit 6 bis 20 Kohlenstoffatomen, eine Acylgruppe mit 2 bis 16 Kohlenstoffatomen

oder eine Aralkylgruppe mit 7 bis 20 Kohlenstoffatomen, wobei die Alkylgruppen wahlweise durch ein oder mehrere Halogenatome, Alkoxygruppen mit 1 bis 12 Kohlenstoffatomen und Acylgruppen mit 2 bis 16 Kohlenstoffatomen substituiert sein können,

(2) der allgemeinen Formel

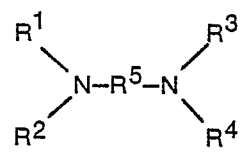

worin

$R^1$, $R^2$, $R^3$, $R^4$, welche gleich oder verschieden sein können, = eine Alkylgruppe mit 1 bis 16 Kohlenstoffatomen, eine Arylgruppe mit 6 bis 20 Kohlenstoffatomen, eine Acylgruppe mit 2 bis 16 Kohlenstoffatomen oder eine Aralkylgruppe mit 7 bis 20 Kohlenstoffatomen, wobei die Alkylgruppen wahlweise durch eine oder mehrere Halogenatome, Alkoxygruppen mit 1 bis 12 Kohlenstoffatomen und Acylgruppen mit 2 bis 16 Kohlenstoffatomen substituiert sein können, und

$R^5$ = eine Alkylengruppe mit 1 bis 8 Kohlenstoffatomen, eine Arylengruppe mit 6 bis 12 Kohlenstoffatomen oder eine Gruppe der Formel

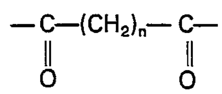

worin n = 2 bis 4,

(3) der allgemeinen Formel

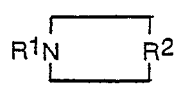 ,

worin

$R^1$ = eine Alkylgruppe mit 1 bis 16 Kohlenstoffatomen, eine Alkylgruppe mit 6 bis 20 Kohlenstoffatomen, eine Acylgruppe mit 2 bis 16 Kohlenstoffatomen oder eine Aralkylgruppe mit 7 bis 20 Kohlenstoffatomen, wobei die Alkylgruppen wahlweise durch ein oder mehrere Halogenatome, Alkoxygruppen mit 1 bis 12 Kohlenstoffatomen und Acylgruppen mit 2 bis 16 Kohlenstoffatomen substituiert sein können, und

$R^2$ =

(A) eine Alkylengruppe mit 3 bis 6 Kohlenstoffatomen,

(B) eine Alkylengruppe mit 3 bis 6 Kohlenstoffatomen, substituiert durch ein oder mehrere Alkylgruppen mit 1 bis 8 Kohlenstoffatomen, Arylgruppen mit 6 bis 20 Kohlenstoffatomen, Aralkylgruppen mit 7 bis 20 Kohlenstoffatomen und Halogenatomen,

(C) eine Formel $-(CH_2)_m-O-(CH_2)_n$, worin m, n = 1 bis 5 und (m + n) = 3 bis 6, oder

(D) eine Formel,

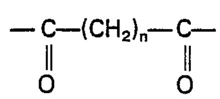

worin n = 2 bis 4,

(4) der allgemeinen Formel

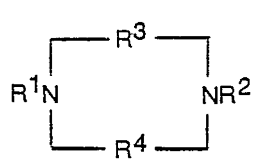

worin

$R^1$, $R^2$, welche gleich oder verschieden sein können, = eine Alkylgruppe mit 1 bis 16 Kohlenstoffatomen, eine Arylgruppe mit 6 bis 20 Kohlenstoffatomen, eine Acylgruppe mit 2 bis 16 Kohlenstoffatomen, eine Aralkylgruppe mit 7 bis 20 Kohlenstoffatomen, wobei die Alkylgruppe wahlweise durch ein oder mehrere Halogenatome, Alkoxygruppen mit 1 bis 12 Kohlenstoffatomen und Acylgruppen mit 2 bis 16 Kohlenstoffatomen substituiert sien kann, und

$R^3$, $R^4$, welche gleich oder verschieden sein können, gleich

(A) eine Alkylengruppe mit 1 bis 6 Kohlenstoffatomen,

(B) eine substituierte Alkylengruppe mit 1 bis 6 Kohlenstoffatomen, die durch eine oder mehrere

Alkylgruppen mit 1 bis 8 Kohlenstoffatomen, Arylgruppen mit 6 bis 20 Kohlenstoffatomen, Acylgruppen mit 2 bis 16 Kohlenstoffatomen, Aralkylgruppen mit 7 bis 20 Kohlenstoffatomen und Halogenatome substituiert ist,
(C) eine Formel, $—(CH_2)_m—O—(CH_2)_n—$, worin m, n = 1 bis 5 und (m + n) = 3 bis 6,
(D) eine Formel,

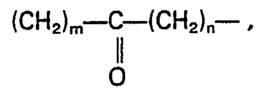

worin m, n = 0 bis 5 und (m + n) = 2 bis 6, oder
(E) eine Formel,

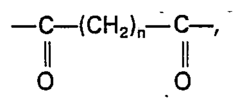

worin n = 2 bis 4,
bedeuten.

2. Verfahren gemäß Anspruch 1, worin das tertiäre Amin die allgemeine Formel $R^1R^2R^3N$ aufweist.

3. Verfahren gemäiß Anspruch 1, worin das tertiäre Amin die allgemeine Formel:

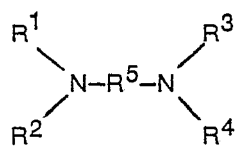

aufweist.

4. Verfahren gemäß Anspruch 1, worin das tertiäre Amin die allgemeine Formel:

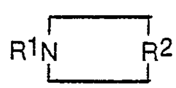

aufweist.

5. Verfahren gemäß Anspruch 1, worin das tertiäre Amin die allgemeine Formel:

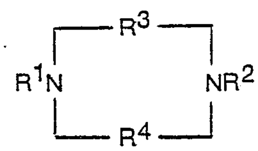

aufweist.

6. Verfahren gemäß einem der Ansprüche 1 bis 5, worin das Ausgangsmaterial ein Isoolefin umfaßt.

**Revendications**

1. Procédé de production d'acétone ou de méthyléthylcétone, qui consiste à oxyder à une température de 40°C à 140°C du propylène ou respectivement du n-butène en présence d'un catalyseur formé d'une solution aqueuse contenant du palladium, du cuivre, un halogène et au moins l'une des amines tertiaires représentées par les formules générales suivantes:

(1) formule générale $R^1R^2R^3N$, dans laquelle

$R^1$ est un groupe aryle ayant 6 à 20 atomes de carbone, un groupe aralkyle ayant 7 à 20 atomes de carbone, ou un groupe alkyle substitué comprenant un groupe alkyle de 1 à 16 atomes de carbone portant un ou plusieurs substituants, obtenu par substitution d'hydrogène dans ledit groupe alkyle avec un atome d'halogène, un groupe alkoxy ayant 1 à 12 atomes de carbone ou un groupe acyle ayant 2 à 16 atomes de carbone et

$R^2$, $R^3$ qui peuvent être égaux ou différents représentent un groupe alkyle ayant 1 à 16 atomes de carbone, un groupe aryle ayant 6 à 20 atomes de carbone, un groupe acyle ayant 2 à 16 atomes de carbone ou un groupe aralkyle ayant 7 à 20 atomes de carbone, le groupe alkyle étant éventuellement substitué par un ou plusieurs atomes d'halogènes, groupes alkoxy ayant 1 à 12 atomes de carbone et groupes acyle ayant 2 à 16 atomes de carbone,

(2) formule générale

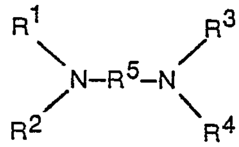

dans laquelle

$R^1$, $R^2$, $R^3$, $R^4$, qui peuvent être égaux ou différents, représentent un groupe alkyle ayant 1 à 16 atomes de carbone, un groupe aryle ayant 6 à 20 atomes de carbone, un groupe acyle ayant 2 à 16 atomes de carbone ou un groupe aralkyle ayant 7 à 20 atomes de carbone, le groupe alkyle étant éventuellement substitué par un ou plusieurs atomes d'halogènes, groupes alkoxy ayant 1 à 12 atomes de carbone et groupes acyle ayant 2 à 16 atomes de carbone, et

$R^5$ est un groupe alkylène ayant 1 à 8 atomes de carbone, un groupe arylène ayant 6 à 12 atomes de carbone ou un groupe

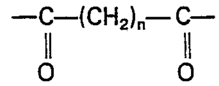

dans leque n a une valeur de 2 à 4,

(3) formule générale

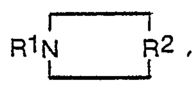

dans laquelle

$R^1$ est un groupe alkyle ayant 1 à 16 atomes de carbone, un groupe aryle ayant 6 à 20 atomes de carbone, un groupe acyle ayant 2 à 16 atomes de carbone ou un groupe aralkyle ayant 7 à 20 atomes de carbone, le groupe alkyle étant éventuellement substitué par un ou plusieurs atomes d'halogènes, groupes alkoxy ayant 1 à 12 atomes de carbone et groupes acyle ayant 2 à 16 atomes de carbone, et

$R^2$ représente

(A) un groupe alkylène de 3 à 6 atomes de carbone,

(B) un groupe alkylène de 3 à 6 atomes de carbone substitués par un ou plusieurs groupes alkyle ayant 1 à 8 atomes de carbone, groupes aryle ayant 6 à 20 atomes de carbone, groupes aralkyle ayant 7 à 20 atomes de carbone, et atomes d'halogènes.

(C) un groupe de formule $-(CH_2)_m-O-(CH_2)_n$, dans lequel m et n ont une valeur de 1 à 5 et la somme m + n a une valeur de 3 à 6, ou bien

(D) un groupe de formule

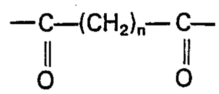

dans laquelle n a une valeur de 2 à 4,

(4) formule générale

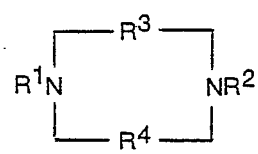

dans laquelle $R^1$, $R^2$, qui peuvent être égaux ou différents, représentent un groupe alkyle ayant 1 à 16 atomes de carbone, un groupe aryle ayant 6 à 20 atomes de carbone, un groupe acyle ayant 2 à 16 atomes de carbone, un groupe aralkyle ayant 7 à 20 atomes de carbone, le groupe alkyle étant éventuellement substitué par un ou plusieurs atomes d'halogènes groupes alkoxy en $C_1$ à $C_{12}$ et groupes acyle en $C_2$ à $C_{16}$ et

$R^3$, $R^4$, qui peuvent être égaux ou différents représentent

(A) un groupe alkylène ayant 1 à 6 atomes de carbone,

(B) un groupe alkylène substitué ayant 1 à 6 atomes de carbone, substitués par un ou plusieurs groupes alkyle ayant 1 à 8 atomes de carbone, groupes aryle ayant 6 à 20 atomes de carbone, groupes acyle ayant 2 à 16 atomes de carbone, groupes aralkle ayant 7 à 20 atomes de carbone, et atomes d'halogènes,

(C) un composé de formule $-(CH_2)_m-O-(CH_2)_n-$ dans laquelle m et n ont valeur de 1 à 5 et la somme (m + n) a une valeur de 3 à 6,

(D) un composé de formule

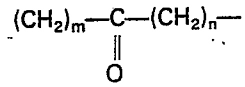

dans laquelle m et n ont une valeur de 0 à 5 et la somme (m + n) a une valeur de 2 à 6, ou bien (E) un composé de formule

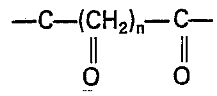

dans laquelle n a une valeur de 2 à 4.

2. Procédé suivant la revendication 1, dans lequel l'amine tertiare répond à la formule générale $R^1R^2R^3N$.

3. Procédé suivant la revendication 1, dans lequel l'amine tertiaire répond à la formule générale

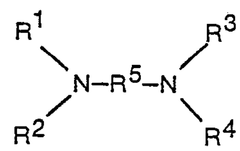

4. Procédé suivant la revendication 1, dans lequel l'amine tertiaire répond à la formule générale

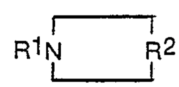

5. Procédé suivant la revendication 1, dans lequel l'amine tertiaire répond à la formule générale

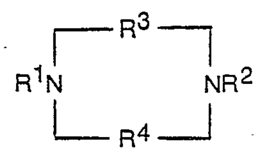

6. Procédé suivant l'une quelconque des revendications 1 à 5, dans lequel la matière de départ comprend une iso-oléfine.